(19) Europäisches Patentamt / European Patent Office / Office européen des brevets

(11) **EP 3 915 544 A1**

(12) **EUROPEAN PATENT APPLICATION**

(43) Date of publication:
**01.12.2021 Bulletin 2021/48**

(51) Int Cl.:
***A61K 9/127*** (2006.01)

(21) Application number: **20176366.1**

(22) Date of filing: **25.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **leon-nanodrugs GmbH**
**81679 München (DE)**

(72) Inventors:
• **Mühlhölzl-Odörfer, Dr. Kathrin**
  **80469 Munich (DE)**
• **Horstkotte, Dr. Elke**
  **81245 Munich (DE)**

(74) Representative: **Held, Stephan**
**Meissner Bolte Patentanwälte**
**Rechtsanwälte Partnerschaft mbB**
**Widenmayerstraße 47**
**80538 München (DE)**

(54) **METHOD FOR PRODUCING A LIPOSOME DISPERSION**

(57) A method for producing a liposome dispersion comprising an active pharmaceutical ingredient and at least one amphiphilic lipid, wherein the method comprises the steps of: providing a first stream (a) comprising an organic solvent, providing a second stream (b) comprising water, wherein at least one of first stream (a) and second stream (b) comprises the at least one amphiphilic lipid, and wherein at least one of first stream (a) and second stream (b) comprises the active pharmaceutical ingredient, and colliding the first stream (a) and the second stream (b), whereby the liposome dispersion comprising the active pharmaceutical ingredient and the at least one amphiphilic lipid is obtained, wherein the hydrodynamic pressure in the first stream (a) and/or in the second stream (b) is around 30 bar or less.

Fig. 1

**Description**

The present invention relates to a method for producing a liposome dispersion. Further, the present invention relates to a liposome dispersion comprising an active pharmaceutical ingredient and at least one lipid, obtainable by a method according to the invention.

Different technologies are used for the production of solid lipid particles incorporating poorly water-soluble active pharmaceutical ingredients and for the production of liposomal products incorporating poorly water-soluble or water-soluble active pharmaceutical ingredients.

It is known that oral bioavailability of poorly soluble active pharmaceutical ingredients can be increased by reducing the particle size of the active pharmaceutical ingredients. Moreover, application of nanotechnology for nanosizing and/or encapsulation of poorly soluble active pharmaceuticals as well as encapsulation of water soluble active pharmaceuticals for e.g. parenteral administration showed advantages in terms of e.g. increase in bioavailability, reduction of side effects, passive targeting to tumor sites by enhanced permeation though leaky junctions, etc. To be emphasized that nanotechnology is used to enable formulation of poorly water soluble active pharmaceutical ingredients for parenteral administration in cases where the required drug concentration to be administered exceeds the aqueous solubility of the pharmaceutical compound and/or the use of co-solving solvents or co-solving agents is not possible.

In nanotechnology for pharmaceutical products, it is furthermore advantageous to be able to set the particle size freely, because passive drug targeting becomes possible. Furthermore, parenteral products must be sterile. Thus, for parenteral products, a small particle size, which allows sterile filtration, is useful. Other types of sterilisation (e.g. autoclaving) are very likely to destroy liposomes and are therefore not used.

In order to reduce the particle size of active pharmaceutical ingredients, EP 2395978 B1 suggests a so called solvent/nonsolvent precipitation. Solvent/nonsolvent precipitation means that a substance is dissolved in a solvent and collides as a liquid jet with a second liquid jet, whereby the dissolved substance is precipitated. The microjet reactor technology creates a highly turbulent mixing zone of solvent and nonsolvent and is therefore particularly suitable for the precipitation of particles in the nanometer range. EP 2395978 B1 describes the solvent/nonsolvent precipitation in the presence of surface-active molecules using a microjet reactor according to EP 1 165 224 B1. Such a microjet reactor has at least two nozzles (pin-holes) located opposite one another, each with an associated pump and feed line for spraying a liquid medium at a common collision point in a reactor chamber enclosed by a reactor housing. Another opening is provided in the reactor housing through which a gas, an evaporating liquid, a cooling liquid, or a cooling gas can be passed to maintain the gas atmosphere in the reactor chamber or for cooling. A further opening is provided for removing the resulting products and excess gas from the reactor chamber. Thus, a gas, an evaporating liquid or a cooling gas is introduced into the reactor chamber through an opening in order to maintain a gas atmosphere inside the reactor or to cool the resulting products, and the resulting products and excess gas are removed from the reactor chamber through this opening by overpressure on the gas inlet side or by underpressure on the product and gas outlet side. If a solvent/nonsolvent precipitation is carried out in such a microjet reactor, for example as described in EP 2 550 092 A1, a dispersion of precipitated particles is obtained.

In contrast to this common application of microjet reactors, it has later been found that emulsions can be formed using the microjet reactor technology. DE 10 2016 101 232 A1 describes a method for producing emulsions in which very small homogeneous oil droplets can be created with a low energy input. This task is solved by pumping two liquid streams of mutually immiscible liquids through separate openings with a defined diameter in order to achieve a flow velocity of the liquid streams of more than 10 m/s and by colliding the liquid streams. One stream comprises an oil, the other stream comprises an aqueous solution of lecithin. In this case, lecithin is used in the aqueous phase in low amounts as emulsifying agent. Due to the collision of the liquid streams with high flow velocities and kinetic energy, a homogeneous emulsion with an oil droplet size of less than 1 $\mu$m is obtained. No further energy input, such as shear forces, is required. Due to the oil droplet size of less than 1 $\mu$m, the resulting emulsion is very stable. According to this publication, the droplet size of the emulsion depends on the nozzle size in the microjet reactor and on the pump pressure of the feeding pumps for the two liquid streams. The pressure of the liquid jets is preferably between 10 and 1,000 bar and more preferably between 20 and 500 bar. According to this publication, the collision energy in the microjet reactor does not cause any precipitation reactions, but results in emulsions.

Surprisingly, the inventors of the present invention have found that the microjet reactor can be used for production of liposomes. In contrast to emulsions, liposomes do not represent a dispersion of oil and water. Liposomes are formed from amphiphilic lipids, such as glycerophospholipids, by dispersion in an aqueous medium. Liposomes are closed structures, often spherical, and composed of curved lipid bilayers, which enclose part of the surrounding aqueous medium into their interior. The size of a liposome ranges from 20 nm up to several micrometers and they may be composed of one or several concentric or nonconcentric bilayer membranes, each with a thickness of about 4 nm. Small liposomes (approximately 25 to 100 nm) generally contain one phospholipid bilayer. Large liposomes (approximately 100 to 1000 nm) generally contain several phospholipid bilayers. Special types of liposomes that are also based on bilayer-forming, amphiphilic lipids include lipid complexes, lipid nanoparticles, and lipoplexes.

The main constituents of liposomes are phospholipids, which are amphiphilic molecules containing water soluble, hydrophilic head section and a lipid-soluble, hydrophobic tail section.

Liposomes have gained extensive attention as carriers for a wide range of drugs due being both nontoxic and biodegradable because they are composed of naturally occurring substances existing in biological membranes. Biologically active materials encapsulated within liposomes are protected to varying extent from immediate dilution or degradation, which makes them good drug delivery systems for the transport of drugs or other bioactive compounds to organs affected. The unique ability of liposomes to entrap drugs both in an aqueous and a lipid phase make such delivery systems attractive for hydrophilic as well as hydrophobic drugs: Liposomes can contain an active pharmaceutical ingredient, which is water-soluble, within water contained in their interior and/or between the bilayers. Further, liposomes can contain an active pharmaceutical ingredient, which is oil-soluble, within their bilayers in dissolved state (Chrai S., Murari R. & Ahmad I. (2002), Liposomes (a Review), Part Two: Drug Delivery Systems. BioPharm, 40-49; Hong M., Lim S., Lee M., Kim Y. & Kim C. (2001), Prolonged Blood Circulation of Methotrexate by Modulation of Liposomal Composition, Drug Delivery, 1(8): 231-237).

The production of liposomal products is complex and the selection of a particular protocol is primarily dictated by the nature of the therapeutic in the liposomal formulation and should ensure preservation of its stability and biological activity during processing. Protocols that necessitate prolonged exposure to organic solvents or high temperature are unsuitable for protein therapeutics. In addition, the production method should maximize drug entrapment in liposomal vesicles. Among the commonly used methods for the preparation of liposomes extrusion techniques are the most used ones. Extrusion techniques have been used to produce small unilamellar vesicles (SUV, 25-100 nm in size consisting of a single lipid bilayer) from multilamellar vesicles (MLV, multiple lipid bilayers are arranged in a concentric onion skin fashion). These techniques typically involve passage of an MLV aqueous liposome dispersion through polycarbonate membranes or filters of definite size. Typically, smaller size vesicles are obtained by the sequential passage of the MLV aqueous liposome dispersion through a series of progressively smaller pore size filters [Patil, S. D., & Burgess, D. J. (2005), Liposomes: Design and Manufacturing, In D. Burgess (Ed.), Injectable Dispersed Systems (Vol. 20053164, pp. 249-303), Informa Healthcare].

There are examples showing that laminar flow mixing devices can be used for the production of liposomes, e.g. the NanoAssemblr® Technology mixing device from Precision NanoSystems.

The production of liposomal products is complex, often an iterative process with several cycles during production. The large-scale implementation of lab-scale processes is difficult. This is time and cost intensive. Therefore, there is a need for an improved process.

The inventors of the present patent application have found that liposome dispersions can be obtained in an one-step continuous process, using a precipitation device with impinging jets as e.g. described in EP 2395978 B1 or WO 2018/234217 A1.

Thus, a first aspect of the present invention is directed to a method for producing a liposome dispersion. The liposome dispersion comprises an active pharmaceutical ingredient and at least one amphiphilic lipid. The method comprises the steps of:

- providing a first stream (a) comprising an organic solvent,

- providing a second stream (b) comprising water,
  wherein at least one of the first stream (a) and the second stream (b) comprises the at least one amphiphilic lipid, and wherein at least one of the first stream (a) and the second stream (b) comprises the active pharmaceutical ingredient, and

- colliding the first stream (a) and the second stream (b), whereby the liposome dispersion comprising the active pharmaceutical ingredient and the at least one amphiphilic lipid is obtained.

The verbs "to comprise" and "to contain" and their conjugations comprise the term "to consist of" and its conjugations. The terms "one" or "a" comprise the term "at least one". Embodiments of the invention may be combined in any manner unless this is clearly not intended. Embodiments of different aspects of the invention may also be combined.

In one embodiment the hydrodynamic pressure in the first stream (a) and/or in the second stream (b) is around 30 bar or less.

In one embodiment, the drug loading of the "liposome dispersion" is below about 30 wt.-%, such as below about 20 wt.-%, or below about 10 wt.-%.

The term "amphiphilic lipid" and compounds encompassed by this term are well known to the person skilled in the art. For example, the term comprises fatty acids, glycerolipids (monoglycerides, diglycerides), glycerophospholipids (phospholipids, phosphatidylcholine, phosphatidylethanolamine, phosphatidylserine), sphingolipids, sterols (cholesterol), prenols (carotenoids, retinol, retinal, retinoic acid, beta-carotene, tocopherol), saccharolipids. Preferably, the amphiphilic lipid is able to form a bilayer in an aqueous medium. Glycerophospholipids, preferably selected from phosphatidylcholines, and/or cholesterol are particularly preferred as amphiphilic lipid. Phosphatidylcholine is particularly preferred as amphiphilic lipid (e.g. LIPOID S 100), optionally in combination with cholesterol.

The active pharmaceutical ingredient preferably is a small molecule respectively a small chemical compound, a biological molecule, like an antibody, a protein, a peptide, a RNA and a DNA. The term active pharmaceutical ingredient comprises also diagnostics, that is substances used in the diagnostic field, like contrast agents, RNA-probes, DNA-probes, labeled inorganic and organic molecules.

In one embodiment, the active pharmaceutical ingredient is a small molecule respectively a small chemical compound.

The active pharmaceutical ingredient may have good solubility in water and/or organic solvents (e.g. octanol).

In one embodiment, the active pharmaceutical ingredient is practically insoluble in water (e.g. < 0.1 mg/ml). In this embodiment, it is preferred that the first stream (a) comprises the active pharmaceutical ingredient. In another embodiment, the active pharmaceutical ingredient is practically insoluble in the organic solvent used (e.g. < 0.1 mg/ml). In this embodiment, it is preferred that second stream (b) comprises the active pharmaceutical ingredient.

Preferably, the active pharmaceutical ingredient, if it is a so-called small molecule, has a molecular weight in the range from about 300 g/mol to about 1,200 g/mol, or in the range from about 400 g/mol to about 1,000 g/mol.

The at least one amphiphilic lipid is preferably soluble in the organic solvent of the first stream (a). Thus, the first stream (a) may comprise the at least one amphiphilic lipid. The solvent in the first stream (a) is an organic solvent. The solvent may be a mixture of several organic solvents. Preferably, the solvent is not the at least one amphiphilic lipid (not the same compound or mixture of compounds).

The main liquid component (e.g. $\geq$ 50, 60, 70, 75, 80, 90, 95, or 99 wt.-%) of the second stream (b) is water. The second stream (b) comprises water and optionally comprises excipients such as surfactants. In other words, the second stream (b) may comprise lipids with amphiphilic properties such as glycerophospholipids, preferably selected from phosphatidylcholines. Thus, the second stream (b) may comprise the at least one amphiphilic lipid.

In one embodiment, the first stream (a) and the second stream (b) comprise the at least one amphiphilic lipid.

The liposome dispersion comprises liposomes. This requires that the first stream (a) comprises at least one amphiphilic lipid, e.g. a glycerophospholipid, and/or that the second stream (b) comprises at least one amphiphilic lipid, e.g. a glycerophospholipid. It is preferred that none of the stream (a) and the second stream (b) comprises high amounts (e.g. more than 50, 30, 20, 10, 5 or 1 wt.-%) of an oil. Oils might be used as additives, where required, to stabilize the liposomal formulation. Thus, preferably, the oil is not employed as solvent. The presence of a liposome dispersion may be confirmed using cryogenic electron microscopy for determining vesicle size, lamellarity and bilayer thickness. On the basis of their size and number of bilayers, liposomes can be classified into one of two categories: (1) multilamellar vesicles (MLV) and (2) unilamellar vesicles. Unilamellar vesicles can also be classified into two categories: (1) large unilamellar vesicles (LUV) with a characteristic size of 100-500 nm and (2) small unilamellar vesicles (SUV) with a particle size of 20-100 nm. In unilamellar liposomes, the vesicle has a single phospholipid bilayer enclosing the aqueous solution whereas multilamellar liposomes are composed of two to multiple bilayers.

Liposomes prepared by different methods may vary considerably in lamellarity. Lamellarity plays a crucial role in defining liposome properties: determining encapsulation efficiency, mediating diffusion rate of encapsulated agents out of liposomes, controlling drug release, penetration, etc. Moreover, lamellarity may have significant effect on the intracellular fate of the drugs delivered by liposomes after cellular uptake. In one embodiment, the produced liposomes are mainly unilamellar. In another embodiment, the produced liposomes are mainly a mixture of uni- and multilamellar liposomes.

The obtained liposome dispersion is an aqueous liquid, comprising liposomes.

Preferably, the obtained liposome dispersion is further processed, preferably into a pharmaceutical composition. This may include one or more steps selected from adjustment of pH, adjustment of osmolality, removal of organic solvent, concentration, terminal sterilization via sterile filtration, filling, lyophilization, etc.

During collision of the first and the second stream, the streams may be considered as impinging liquid jets. In general, the hydrodynamic pressure for the impinging jets used during production can be either calculated on a theoretic basis or monitored using a pressure meter.

The hydrodynamic pressure calculation for the (impinging) streams of the first stream (a) and the second stream (b) uses the following formula

$$\mathrm{p} = \left( \frac{1}{2} * \rho * \left[ \frac{Q}{\pi * r^2} \right]^2 \right) * 10^{-5}$$

wherein

p = pressure in the stream ([bar])
$\rho$ = density of the composition (all constituents) of the stream ([kg/m$^3$])
Q = flow rate of the stream ([m$^3$/s])
r = radius pin hole ([m]).

Wherein the following term represents the stream velocity v in m/s

$$v = \left[\frac{Q}{\pi * r^2}\right]^2$$

The flow rate is one of the variables for pressure calculation. Preferably, it is ensured that the pumps are functional and meet the required pumping capacity prior to start of the production process. The flow rate can be checked - without using the microjet reactor - by means of pump calibration: for each pump a defined flow rate is set, pumping is executed over time x and pumped volume is checked for weight by using a balance. Experimental and theoretical weights are compared.

In-line monitoring of flow rates can be performed by implementation of flow meters in the feeding lines to the MicroJetReactor, so that the flow can also be confirmed during the process. CORI FLOW Meter or Coriolis Flow Meter from Bronkhorst can be used for this purpose.

Based on the mathematic relation of hydrodynamic pressure and jet velocity, also the jet velocity of the first stream (a) and the second stream (b) - determined by pin hole radius and flow rate - is preferably below 70 m/s, preferably below 50 m/s, preferably below 25 m/s, preferably below 10 m/s, respectively.

A prepared feature of the invention is the hydrodynamic pressure resp. the jet velocity of the impinging jets. As explained above, the hydrodynamic pressure is calculated from the flow rate and density of the streams and from the radius of the pin holes through which the streams are ejected. In order to achieve a hydrodynamic pressure according to the invention, it is generally preferred that the flow rate of first stream (a) is 1 ml/min to 10 l/min, more preferably 1 ml/min to 1.33 l/min, most preferably 4 ml/min to 250 ml/min, the flow rate of second stream (b) is 1 ml/min to 10 l/min, more preferably 1 ml/min to 1.33 l/min, most preferably 4 ml/min to 250 ml/min. It is preferred that the jet velocity of first stream (a) is 0.5 to 70 m/s, preferably 0.5 to 25 m/s, the density of first stream (a) is 0.600 to 1.300 g/mL, and the jet velocity of second stream (b) is 0.5 to 70 m/s, preferably 0.5 to 25 m/s, and that the density of second stream (b) is 0.600 to 1.300 g/mL. It is preferred that the diameter (radius = half of diameter) of the pin-holes is 5 to 5,000 $\mu$m, preferably 5 to 3,000 $\mu$m, more preferably 10 to 2,000 $\mu$m, and most preferably 50 to 500 $\mu$m.

Preferred embodiments of the invention are included in the claims.

The hydrodynamic pressure in the (impinging) first stream (a) is preferably about $\leq$ 30 bar, more preferably about $\leq$ 25 bar, about $\leq$ 12 bar, or even about $\leq$ 5 bar. In other words, the hydrodynamic pressure in the first stream (a) is preferably from about 0.0001 bar to about 30 bar, preferably from about 0.001 bar to about 25 bar, or from about 0.01 bar to about 12 bar, or from about 0.01 bar to about 5 bar.

The hydrodynamic pressure in the (impinging) second stream (b) is preferably about $\leq$ 30 bar, more preferably about $\leq$ 25 bar, or about $\leq$ 12 bar, or about $\leq$ 5 bar. In other words, the hydrodynamic pressure in the second stream (b) is preferably from about 0.0001 bar to about 30 bar, preferably from about 0.001 bar to about 25 bar, or from about 0.01 bar to about 12 bar, or from about 0.01 bar to about 5 bar.

Preferably, the hydrodynamic pressure in the first stream (a) is equal to or lower than the hydrodynamic pressure in the second stream (b).

Preferably, the first stream (a) comprises the at least one amphiphilic lipid. Preferably, the first stream (a) comprises the at least one amphiphilic lipid, wherein the first stream (a) comprises from 1 to 500 mg/mL, or from 25 to 250 mg/mL, or from 50 to 200 mg/mL, of glycerophospholipids, preferably selected from phosphatidylcholines. In other words, preferably, the at least one amphiphilic lipid comprises glycerophospholipids, preferably selected from phosphatidylcholines.

Preferably, the first stream (a) and/or the second stream (b) comprises from 0.1 to 250 mg/mL, preferably from 1 to 100 mg/mL, more preferably from 5 to 50 mg/mL, of the active pharmaceutical ingredient.

The content of the at least one amphiphilic lipid and the active pharmaceutical ingredient in the first stream (a) and/or the second stream (b) can be important for the liposome dispersion formation, stabilization, and release of the active pharmaceutical ingredient.

In one embodiment, the first stream (a) comprises 1 to 500 mg/mL, or from 5 to 250 mg/mL, or from 5 to 100 mg/mL, of cholesterol. Cholesterol may influence liposome dispersion formation, stabilize the liposome dispersion, and influence release of the active pharmaceutical ingredient.

In one embodiment, the first stream (a) comprises a steroid, preferably cholesterol or its derivative, preferably from 1 to 500 mg/mL, or from 5 to 250 mg/mL, or from 5 to 100 mg/mL.

In one embodiment, the first stream (a) and/or the second stream (b) comprises from 0.5 to 250 mg/mL, or from 0.5 to 50 mg/mL, or from 0.5 to 10 mg/ml of one or more (ionic or non-ionic solvent soluble resp. nonsolvent soluble) surfactants, preferably selected from polyethoxylated castor oil (Kolliphor ELP), Macrogol 15 Hydroxystearat (Kolliphor HS15), pegylated castor oils, Docusate Sodium, Poly(ethylene glycol)-*block*-poly(propylene glycol)-*block*-poly(ethylene glycol) (Poloxamer 188), sodium oleate, oleic acid, sodium deoxycholate, deoxycholic acid, Vitamine E and Vitamine E derivatives, polysorbate 20 (Tween 20), polysorbate 40 (Tween 40), polysorbate 60 (Tween 60), polysorbate 80 (Tween 80). Surfactant may influence liposome dispersion formation, stabilize the liposome dispersion, and influence release of the active pharmaceutical ingredient.

In one embodiment, the first stream (a) comprises from 0.5 to 250 mg/mL, or from 0.5 to 50 mg/mL, or from 0.5 to 10 mg/mL, of one or more ionic or non-ionic surfactants.

In one embodiment, the second stream (b) comprises from 0.5 to 250 mg/mL, or from 0.5 to 50 mg/mL, or from 0.5 to 10 mg/mL, of one or more ionic or non-ionic surfactants.

When colliding the streams, the streams are preferably arranged towards each other at an angle of approximately 180 °C. In other words, the streams are preferably collided frontally.

Preferably, in the colliding step, the volume ratio of the first stream (a) to the second stream (b) is between 50:50 to 4:96, more preferably of between 30:70 to 9:91.

In a preferred embodiment, a microjet reactor is used to carry out the method. Such a microjet reactor is known from EP 1 165 224 B1. Other micromixing devices and/or precipitation devices with impinging jets may also be used, e.g. a device as described in WO 2018/234217 A1.

Preferably, the distance between opposing pin-holes is less than 5 cm, preferably less than 3 cm, and more preferred less than 1 cm.

The reactor chamber can be filled with gas. Gas, especially inert gas or inert gas mixtures, but also reactive gas can be supplied through a gas inlet into the reactor chamber. It is preferred that the gas pressure in the reactor chamber is 0.05 to 30 bar, preferably 0.2 to 10 bar, and particularly preferably 0.5 to 5 bar. Preferably, the process can be executed without any gas other than air.

Preferably, the method is a continuous method.

Preferably, the solvent of the first stream (a) comprises or is an organic solvent, which is miscible with water. The solvent of the first stream (a) can also comprise a mixture of 2 or more organic solvents. In one embodiment, the solvent of the first stream (a) comprises ethanol. Preferably, the solvent of the first stream (a) comprises an organic solvent, which is miscible with water, wherein more preferably the solvent of the first stream (a) comprises i) ethanol or ii) a mixture of ethanol with a further organic water-miscible and ethanol miscible solvent, wherein most preferably the solvent of the first stream (a) comprises a mixture of ethanol and the further organic solvent with a ratio of ethanol to the further organic solvent between 10:90 to 90:10. The second solvent may be acetone.

Preferably, the liposome dispersion has an average particle size of from 1 to 1000 nm, preferably of from 5 to 500 nm, more preferably of from 10 to 300 nm, or of from 25 to 200 nm.

Preferably, the liposome dispersion is monodisperse with a polydispersity index from 0.1 to 0.4, preferably below 0.3, such as around 0.20.

The method of the invention can result in a liposome dispersion within the above size ranges and/or polydispersity index.

The term "average particle size", when used herein to describe the size of liposomes, refers to the z-average diameter. Throughout this document, the z-average diameter is measured by Dynamic Light Scattering (e.g. using a Malvern Zetasizer ZS90 or ZS from Malvern Instruments Ltd.) at a temperature of 25 C°. This temperature is kept constant during the measurement. The z-average diameter, together with the polydispersity index (PDI), is preferably calculated from the cumulants analysis of the DLS measured intensity autocorrelation function as defined in ISO22412:2008. PDI is a dimensionless estimate of the width of the particle size distribution, scaled from 0 to 1.

In one embodiment, the liposome dispersion is subjected to sterile filtration. Preferably, the filter material is selected from cellulose acetate, regenerated cellulose, polyamide, polyethersulfone (PES), modified polyethersulfone (mPES), polyvinylidene fluoride (PVDF) and polytetrafluoroethylene (PTFE). Sterile filtration allows parenteral application of the product and is method of choice - according to regulatory requirements - for products for parenteral use that cannot be terminally sterilized by moist heat or dry heat. Thanks to the method of the invention, the particle size can be set. This is important, because the loss of product can be minimized during filtration by choosing a particle size below 150 nm with a low PDI (and by choosing an appropriate filter material).

In one embodiment, the liposome dispersion is subjected to lyophilization. Lyophilization may increase stability of the liposome dispersion.

In one embodiment, the organic solvents used for production of the liposome dispersion can be removed via a cross-flow filtration process. In one embodiment, the organic solvents used for production of the liposome dispersion are removed via a cross-flow filtration process.

In a second aspect, the invention is directed to a liposome dispersion comprising an active pharmaceutical ingredient and at least one amphiphilic lipid, wherein the liposome dispersion is obtainable by any method according to the invention. In a third aspect, the invention is directed to a pharmaceutical composition comprising the liposome dispersion according to the invention.

Preferably, the pharmaceutical composition is a pharmaceutical composition for parenteral or oral application, preferably for parenteral application.

In one embodiment, the pharmaceutical composition is a liposomal formulation comprising the liposome dispersion of the invention.

In one embodiment, the pharmaceutical composition is a lyophilisate for reconstitution before application.

In one embodiment, the pharmaceutical composition can be / has been terminally sterilized by filter sterilization.

The invention will now be described using non-limiting examples.

Formulations 1, 2, and 3 were prepared according to the invention.

The active pharmaceutical ingredient that was used for these examples represents a small molecule, that is practically insoluble in water (<0.1 mg/ml), with a molecular weight of 450 g/mol.

The drug loading can be taken from the following examples.

It is assumed that the lipophilic active ingredient is incorporated in the lipid bilayer of a liposome. This allows the formulation of poorly water soluble substances for parenteral administration, either as a reconstitution product in case of stability problems or as ready-to-use liposome formulation. Water soluble substances can be incorporated in dissolved state in the aqueous interior of the liposomes.

Example 1

Formulation 1 was prepared according to a method of the invention. The method comprised the steps of:

- providing a first stream (a) comprising an organic solvent, the active pharmaceutical ingredient, and the at least one amphiphilic lipid,
- providing a second stream (b) comprising water, and
- colliding the first stream (a) and the second stream (b) using a microjet reactor, whereby the liposome dispersion comprising the active pharmaceutical ingredient and the at least one amphiphilic lipid was obtained.

First stream (a) comprised a mixture of 3:1 (v/v) ethanol/acetone as solvent.

The above conditions were identical for formulations 1, 2, and 3.

For formulation 1, the concentration of active pharmaceutical ingredient in first stream (a) was 12.5 mg/ml. First stream (a) comprised as the at least one amphiphilic lipid 200 mg/ml Lipoid S100 (a soybean phosphatidylcholine ($\geq$ 94 %)) and 10 mg/ml cholesterol. Further, first stream (a) comprised 6.25 mg/ml polyethoxylated castor oil. The second stream (b) consisted of water.

For formulation 1, the hydrodynamic pressure in the first stream (a) was 0.07 bar and the hydrodynamic pressure in the second stream (b) was 2.25 bar. The pinhole diameters used were 500 $\mu$m. Flow rates were 50 ml/min for first stream (a) and 250 ml/min for second stream (b). No gas flushing was used for production.

Values for jet velocity as well as hydrodynamic pressures are given in Table 1:

Table 1.

| Stream | Pin hole diameter / $\mu$m | Flow rate / ml/min | Jet velocity / m/s | Density / kg/m3 | Hydrodyna mic pressure / bar |
|---|---|---|---|---|---|
| (a) | 500 | 50 | 4.2 | 800 | 0.07 |
| (b) | 500 | 250 | 21.2 | 1000 | 2.25 |

Aqueous liquids, each comprising liposomes were obtained. Liposome size given as z-average as well as PDI are given in Table 2. For size analyses liposome dispersions were diluted as follows: 20 $\mu$l liposome dispersion ad 1 ml water.

Table 2.

| Sub-batch | API / mg/mL | Particle size / nm | PDI |
|---|---|---|---|
| 1 | 2.0 | 141.0 | 0.34 |
| 2 | 2.1 | 140.1 | 0.35 |

(continued)

| Sub-batch | API / mg/mL | Particle size / nm | PDI |
|---|---|---|---|
| 3 | 2.0 | 142.6 | 0.38 |
| 4 | 2.0 | 148.7 | 0.33 |

Calculated liposome dispersion and liposome composition are given in Table 3:

Table 3.

| | API | Lipoid S100 | Cholesterol | Polyethoxylat ed castor oil |
|---|---|---|---|---|
| Composition in liposome dispersion / mq/ml | 2.1 | 33.3 | 1.7 | 1.0 |
| % liposome composition | 5.6 | 89.9 | 4.5 | - |

Example 2

A pharmaceutical composition according to Example 1 was produced. A cross-flow filtration (CFF) process was used for removal of organic solvents and dissolved active pharmaceutical ingredient as well as excipients. At the end of the process concentration of the liposome dispersion was performed. A lab-scale set-up was used with a peristaltic pump feeding the liposome dispersion to a mPES 50 kDa hollow fiber filter at a pressure of 30 psi (2 bar). Seven volume exchanges were performed using a wash solution of water containing surfactant (1.2 mg/ml polyethoxylated castor oil) as cross-flow filtration medium. Concentrated aqueous liquid was pooled after CFF and adjusted for osmolality with 5 % mannitol and for pH with NaOH. The aqueous liquid comprised liposomes.

Table 4 shows the concentration of active pharmaceutical ingredient, particle size, and PDI before and after CFF for four sub-batches:

Table 4.

| Sub-batch | Before CFF | | | After CFF | | |
|---|---|---|---|---|---|---|
| | API / mg/mL | Particle size / nm | PDI | API / mg/mL | Particle size / nm | PDI |
| 1 | 2.0 | 141.0 | 0.34 | 5.9 | 155.4 | 0.216 |
| 2 | 2.1 | 140.1 | 0.35 | 6.0 | 157.1 | 0.241 |
| 3 | 2.0 | 142.6 | 0.38 | 6.1 | 166.0 | 0.225 |
| 4 | 2.0 | 148.7 | 0.33 | 6.3 | 166.2 | 0.198 |

Example 3

A pharmaceutical composition according to Example 1 and 2 was produced. Furthermore, the liposome dispersion was filtered through a sterile filter (0.22 μm). The experiments show that the liposome size and the narrow size distribution is suitable for setting up an economical filtration process with limited or no loss of active pharmaceutical ingredient (API), depending on the filter material used (Table 5):

Table 5: Sterile filtration experiments for formulation 1 using different filter membrane materials (PES = polyethersulfone; PTFE = polytetrafluoroethylene; Nylon = polyamide; RC = regenerated cellulose; CA = cellulose acetate).

| ID | Filter | Pre-filtration | | Post-filtration | | API / mg/mL | | Recovery / % |
|---|---|---|---|---|---|---|---|---|
| | | Particle size / nm | PDI | Particle size / nm | PDI | Pre-filtration | Post-filtration | |
| Formulation 1 | PES | 130.6 | 0.20 | 138.4 | 0.22 | 1.93 | 0.81 | 41.9 |
| | PTFE | | | 130.6 | 0.19 | | 1.16 | 60.3 |
| | Nylon | | | 138.4 | 0.25 | | 1.53 | 79.4 |
| | RC | | | 144.0 | 0.25 | | 1.93 | 100.0 |
| | CA | | | 145.5 | 0.21 | | 1.80 | 93.5 |

Example 4

The obtained carrier system produced according to Examples 1 to 3 is an aqueous liquid and comprises liposomes as the following cryo TEM analysis demonstrates.

The product was mixed prior to taking samples by careful shaking. Samples were diluted with HPLC water containing 1.2 mg/ml polyethoxylated castor oil for analyses at 2 to 3-fold dilution for cryo TEM.

For cryo-TEM examination the samples were vitrified using a Vitrobot (FEI) plunging device. Five $\mu$l of the sample dispersion was applied to a Quantifoil or a holey carbon coated TEM grid that has been glow discharged shortly before. After removing excess sample solution with a filter paper the grid is immediately plunged into liquid ethane. For the subsequent examination this specimen is transferred to a TEM (FEI Tecnai F20) keeping cryogenic conditions using a cryo TEM holder (Gatan 926). Conventional TEM imaging was done using an acceleration voltage of 200 kV. Micrographs were acquired with a 4k Direct Electron Detection Camera (Gatan K2 summit) under low dose conditions.

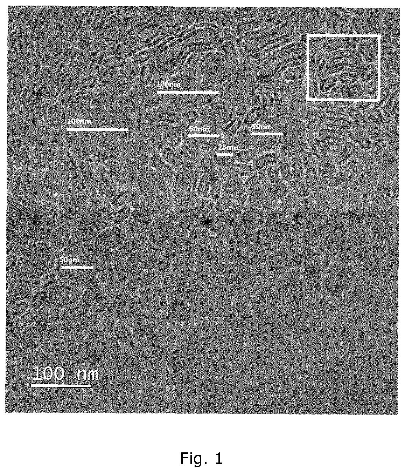

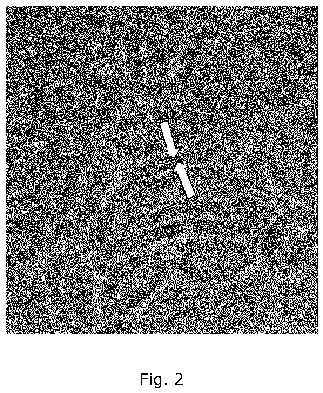

Cryo TEM analysis of the obtained aqueous liquid showed double layer liposome structures - unilamellar as well as multilamellar - varying in size between 25 and 100 nm (see Fig. 1). No signs of crystalline active pharmaceutical ingredient could be observed in intact vesicles. The bilayer structure comprised two lines of low-intensity counting lines (dark) and one inner line with high intensity (white). The thickness of the liposomal bilayer structure was determined as 5 to 7 nm. Knowing that the active pharmaceutical ingredient shows limited water solubility of below 0.1 mg/ml, understanding is that active pharmaceutical ingredient is embedded / dissolved in the phospholipid membrane of the liposomes. The assumption is that the approximately 2 nm broad light zone of the bilayer structure might be a result of the incorporation of the active pharmaceutical ingredient in the bilayer structure (see Fig. 2). In summary, the Cryo TEM analysis confirms that the aqueous liquid comprises liposomes.

10-week old liposome dispersion used for Cryo TEM analysis showed no indices for physical instability upon storage and shipment. In other words, no signs of precipitation, sedimentation or crystallization could be observed by visual examination.

Example 5

Using a pharmaceutical composition according to Example 1 it could be shown that liposome size can be influenced by applying a flow rate as shown in Table 6. Higher flow rates resulted in reduction of mean liposome size. No gas flushing was used for production.

Table 6: Liposome size given as z-average and PDI using different pin hole and flow rate set-ups (density solvent 800 kg/m$^3$, density non-solvent 1000 kg/m$^3$).

| ID | Solvent line (first stream a) | | | | Non-solvent line (second stream b) | | | | Particle size / nm | PDI |
|----|----------------------|-----------------|------------------|------------------|----------------------|-----------------|------------------|------------------|-----------|------|
|    | Pin hole diameter / $\mu$m | Flow rate / ml/min | Velocity / m/s | Pressure / bar | pin hole diameter / $\mu$m | Flow rate / ml/min | Velocity / m/s | Pressure / bar | | |
| 1 | 500 | 10 | 0.9 | 0.003 | 500 | 50 | 4.2 | 0.090 | 259 | 0.38 |
| 2 | 500 | 20 | 1.7 | 0.012 | 500 | 100 | 8.5 | 0.360 | 235 | 0.25 |
| 3 | 500 | 35 | 3.0 | 0.035 | 500 | 175 | 14.9 | 1.103 | 169 | 0.21 |
| 4 | 500 | 50 | 4.2 | 0.072 | 500 | 250 | 21.2 | 2.252 | 145 | 0.34 |
| 5 | 300 | 2 | 0.5 | 0.001 | 300 | 10 | 2.4 | 0.028 | 255 | 0.36 |
| 6 | 300 | 4 | 0.9 | 0.004 | 300 | 20 | 4.7 | 0.111 | 220 | 0.27 |
| 7 | 300 | 8 | 1.9 | 0.014 | 300 | 40 | 9.4 | 0.445 | 209 | 0.25 |

Example 6

Using a pharmaceutical composition according to Example 1 it could be shown that production is reproducible as shown by particle size analysis for different lab-scale production batches (Table 7). Process parameters as described in Example 5/ID4 were used.

Table 7.

| Production | Mean particle size / nm | PDI |
|---|---|---|
| 1 | 145 | 0.34 |
| 2 | 132 | |
| 3 | 131 | 0.24 |
| 4-1 | 134 | 0.32 |
| 4-2 | 131 | 0.31 |
| 5-1 | 132 | 0.28 |
| 5-2 | 129 | 0.25 |
| 5-3 | 131 | 0.26 |
| 6-1 | 132 | 0.32 |
| 6-2 | 132 | 0.28 |
| 6-3 | 133 | 0.28 |
| 7-1 | 139 | 0.31 |
| 7-2 | 140 | 0.29 |
| 8-1 | 141 | 0.34 |
| 8-2 | 140 | 0.35 |
| 8-3 | 143 | 0.38 |
| 8-4 | 149 | 0.33 |
| mean | 136 | 0.30 |
| SD | 5.8 | 0.04 |

Example 7

A pharmaceutical composition according to Example 1 was used and produced applying higher pressure ranges than those described in Example 1 and 5. Process parameters are listed in Table 8. No gas flushing was used for production.

Table 8.

| ID | Solvent line (First stream a) | | | | Non-solvent line (second stream b) | | | | | |
|----|--------------------------------|-----------------------|-----------------|--------------------|--------------------------------|-----------------------|-----------------|--------------------|------------------------|------|
|    | Pin hole diameter / μm | Flow rate / ml/min | Velocity / m/s | Pressure / bar | Pin hole diameter / μm | Flow rate / ml/min | Velocity / m/s | Pressure / bar | Particle size / nm | PDI |
| 8  | 200 | 8.0  | 4.2 | 0.07 | 200 | 50.0 | 26.5 | 3.52  | 184 | 0.25 |
| 9  | 200 | 16.2 | 8.5 | 0.29 | 200 | 92.2 | 48.9 | 11.97 | 131 | 0.26 |
| 10 | 200 | 4.1  | 2.1 | 0.02 | 100 | 33.0 | 70.0 | 24.52 | 133 | 0.26 |

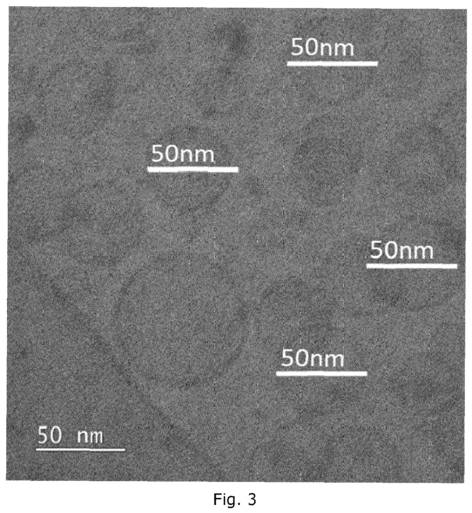

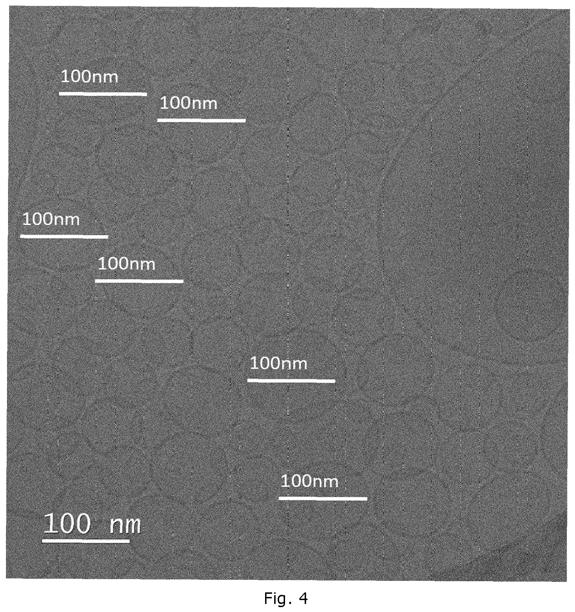

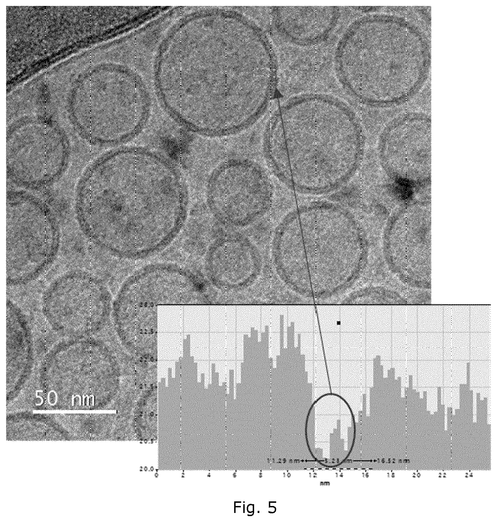

The aqueous liquids ID 8 to 10 comprising solvent and non-solvent - no solvent removal step was applied - were assessed by cryo TEM analysis. Freezing of samples took place the day after production. For all liquids assessed vesicular structures could be observed (Fig. 3 to 5).

Fig. 3 shows a representative cryo TEM section of ID8 with unilamellar vesicles.

Fig. 4 shows a representative cryo TEM section of ID9 with unilamellar vesicles.

Fig. 5 shows a representative cryo TEM section of ID10. Lipid double layers are visible and can be visualized by membrane intensity measurements. The areas with low intensity (low y-axis values) marked by the circle represent the bilayer. A membrane thickness of around 5 nm was assessed exemplary.

Example 8

Formulations 2 and 3 were in general prepared as described for formulation 1. In contrast, the concentration of active pharmaceutical ingredient in first stream (a) was 25 mg/ml. First stream (a) comprised as the at least one amphiphilic lipid 50 mg/ml Lipoid S100 (soybean phosphatidylcholine ($\geq$ 94 %)) and 17.5 mg/ml cholesterol. The second stream (b) contained water and 15 mg/ml Tween 80 (formulation 2) or Tween 20 (formulation 3).

The hydrodynamic pressure in the first stream (a) was 0.01 bar and the hydrodynamic pressure in the second stream (b) was 0.45 bar. The pinhole diameters were 300 $\mu$m. The flow rate used was 8 ml/min for first stream (a) and 40 ml/min for second stream (b).

Formulations 2 and 3 were liposome dispersions. For formulation 2, the z-average particle size was 122 nm and the PDI 0.16. For formulation 3, the z-average particle size was 103 nm and the PDI 0.12.

Calculated liposome dispersion composition and liposome composition for formulation 2 is given in Table 9:

Table 9.

| | | API | Lipoid S100 | Cholesterol | Tween 80 |
|---|---|---|---|---|---|
| Formulation 2 | Composition in liposome dispersion / mq/ml | 4.2 | 8.3 | 2.9 | 12.5 |
| | % liposome composition | 27.0 | 54.1 | 18.9 | - |

Calculated composition of liposome dispersion and liposome composition for formulation 3 is given in Table 10:

Table 10.

| | | API | Lipoid S100 | Cholesterol | Tween 20 |
|---|---|---|---|---|---|
| Formulation 3 | Composition in liposome dispersion / mq/ml | 4.2 | 8.3 | 2.9 | 12.5 |
| | % liposome composition | 27.0 | 54.1 | 18.9 | - |

Example 9

Using a pharmaceutical composition according to Example 8 (formulation 2) it could be shown that production is reproducible as shown by particle size analysis for different lab-scale production batches (Table 11).

Table 11.

| Production | Mean particle size / nm | PDI |
|---|---|---|
| 1 | 129 | 0.16 |
| 2-1 | 127 | 0.22 |
| 2-2 | 138 | 0.22 |
| 2-3 | 127 | 0.25 |
| mean | 130 | 0.21 |
| SD | 4.3 | 0.03 |

Example 10

Formulation 4 was in general prepared as described for formulation 1. In contrast, the concentration of active pharmaceutical ingredient in first stream (a) was 50 mg/ml. First stream (a) comprised as the at least one amphiphilic lipid 10 mg/ml Lipoid S100 (soybean phosphatidylcholine ($\geq$ 94 %)) and 17.5 mg/ml cholesterol. The second stream (b) contained water and 10 mg/ml polyethoxylated castor oil.

Formulations were produced in a similar way as described in Example 7. Process parameters are listed in Table 12. No gas flushing was used for production.

Table 12.

| | | Solvent line (first stream a) | | | Non-solvent line (second stream b) | | | | | |
|---|---|---|---|---|---|---|---|---|---|---|
| ID | pin hole diameter / $\mu$m | Flow rate / ml/min | Velocity / m/s | P / bar | pin hole diameter / $\mu$m | Flow rate / ml/min | Velocity / m/s | P / bar | Particle size / nm | PDI |
| 11 | 200 | 7.3 | 3.9 | 0.06 | 200 | 46.4 | 24.6 | 3.04 | 90 | 0.13 |
| 12 | 200 | 12.3 | 6.5 | 0.17 | 200 | 84.4 | 44.8 | 10.02 | 85 | 0.13 |
| 13 | 200 | 5.4 | 2.8 | 0.03 | 100 | 32.5 | 69.1 | 23.84 | 91 | 0.35 |

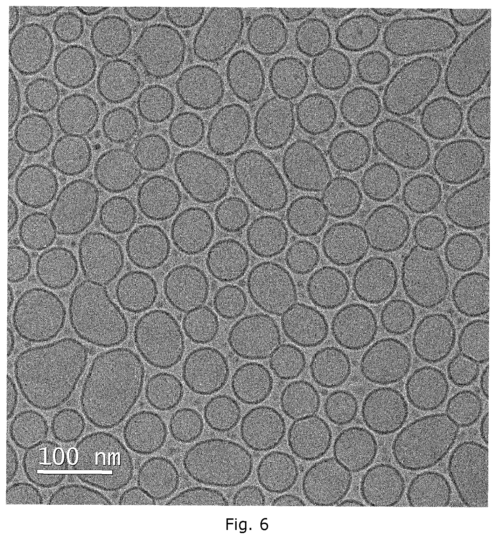

The aqueous liquids ID 11 to 13 comprising solvent and non-solvent - no solvent removal step was applied - were assessed by cryo TEM analysis. Freezing of samples took place the day after production. An exemplary cryo TEM picture for ID 12 is given in Figure 6.

**Claims**

1. A method for producing a liposome dispersion comprising an active pharmaceutical ingredient and at least one amphiphilic lipid, wherein the method comprises the steps of:

   - providing a first stream (a) comprising an organic solvent,
   - providing a second stream (b) comprising water,
   wherein at least one of the first stream (a) and the second stream (b) comprises the at least one amphiphilic lipid, and wherein at least one of the first stream (a) and the second stream (b) comprises the active pharmaceutical ingredient, and
   - colliding the first stream (a) and the second stream (b), whereby the liposome dispersion comprising the active pharmaceutical ingredient and the at least one amphiphilic lipid is obtained.

2. A method at least according to claim 1, **characterized in that** the hydrodynamic pressure in the first stream (a) and/or in the second stream (b) is around 30 bar or less.

3. A method at least according to claim 1 or 2,
   **characterized in that**
   the hydrodynamic pressure in the first stream (a) is about 25 bar or less, or about 12 bar or less, or about 5 bar or less.

4. A method at least according to any of the preceding claims,
   **characterized in that**
   the hydrodynamic pressure in the second stream (b) is about 25 bar or less, or about 12 bar or less, or about 5 bar or less.

5. A method at least according to any of the preceding claims,
   **characterized in that**
   the hydrodynamic pressure in the first stream (a) is lower than or equal to the hydrodynamic pressure in the second stream (b).

6. A method at least according to any of the preceding claims,
   **characterized in that**

the first stream (a) comprises the at least one amphiphilic lipid.

7. A method at least according to any of the preceding claims,
   **characterized in that**
   the first stream (a) comprises from 1 to 500 mg/mL, or from 25 to 250 mg/mL, or from 50 to 200 mg/mL, of glycerophospholipids, preferably selected from phosphatidylcholines.

8. A method at least according to any of the preceding claims, in particular according to claim 6,
   **characterized in that**
   the first stream (a) comprises from 1 to 500 mg/mL, or from 5 to 250 mg/mL, or from 5 to 100 mg/mL of a steroid, preferably of cholesterol or its derivative.

9. A method at least according to any of the preceding claims,
   **characterized in that**
   the first stream (a) and the second stream (b) are collided in a volume ratio of the first stream (a) to the second stream (b) of between 50:50 to 4:96, more preferably of between 30:70 to 9:91.

10. A method at least according to any of the preceding claims,
    **characterized in that**
    the first stream (a) comprises from 0.5 to 250 mg/mL, or from 0.5 to 50 mg/mL, or from 0.5 to 10 mg/mL, of one or more ionic or non-ionic surfactants, and/or the second stream (b) comprises from 0.5 to 250 mg/mL, or from 0.5 to 50 mg/mL, or from 0.5 to 10 mg/mL, of one or more ionic or non-ionic surfactants.

11. A method at least according to any of the preceding claims,
    **characterized in that**
    the solvent of the first stream (a) comprises an organic solvent, which is miscible with water, wherein preferably the solvent of the first stream (a) comprises i) ethanol or ii) a mixture of ethanol with a further organic water-miscible and ethanol miscible solvent, wherein most preferably the solvent of the first stream (a) comprises a mixture of ethanol and the further organic solvent with a ratio of ethanol to the further organic solvent between 10:90 to 90:10.

12. A method at least according to any of the preceding claims,
    **characterized in that**
    the liposome dispersion has an average particle size of from 1 to 1000 nm, preferably of from 5 to 500 nm, more preferably of from 10 to 300 nm, or of from 25 to 200 nm.

13. A method at least according to any of the preceding claims,
    **characterized in that**
    the polydispersity index of the liposome dispersion is from 0.1 to 0.4, preferably below 0.3, such as around 0.20.

14. A method at least according to any of the preceding claims,
    **characterized in that**
    the method further comprises at least one step selected from:

    (i) purifying the liposome dispersion by (sterile) filtration;
    (ii) subjecting the liposome dispersion to lyophilization; and
    (iii) removing organic solvent(s) from the liposome dispersion by cross-flow filtration.

15. A liposome dispersion comprising an active pharmaceutical ingredient and at least one amphiphilic lipid, wherein the liposome dispersion is obtainable by any method according to claims 1 to 14.

Fig. 1

Fig. 2

Fig. 3

Fig. 4

Fig. 5

Fig. 6

Europäisches
Patentamt
European
Patent Office
Office européen
des brevets

# EUROPEAN SEARCH REPORT

**Application Number**

EP 20 17 6366

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/152200 A1 (CUREPORT INC [US]) 25 September 2014 (2014-09-25) * paragraph [0002]; figure 2; examples 1-2 * * paragraphs [0036] - [0038], [0048] * * paragraphs [0056], [0010] - [0013]; figures 2-3 * | 1-15 | INV. A61K9/127 |
| X | US 2005/112184 A1 (JAHN ANDREAS [US] ET AL) 26 May 2005 (2005-05-26) * paragraphs [0018], [0016], [0022]; claims 1-16; figure 2; example 1 * | 1-4, 6-12,14, 15 | |
| X | WO 2016/141161 A1 (CUREPORT INC [US]) 9 September 2016 (2016-09-09) * paragraphs [00117], [00124]; figures 1-2; example 1; table 1 * | 1-15 | |
| X | US 2015/209283 A1 (POLISKY BARRY A [US] ET AL) 30 July 2015 (2015-07-30) * paragraph [0019] * * paragraph [0011] * * paragraphs [0083] - [0092]; example 1 * * claims 1-18; figure 2 * | 1-15 | TECHNICAL FIELDS SEARCHED (IPC) A61K |
| A | WAGNER A ET AL: "THE CROSSFLOW INJECTION TECHNIQUE: AN IMPROVEMENT OF THE ETHANOL INJECTION METHOD", JOURNAL OF LIPOSOME RESEARCH, TAYLOR & FRANCIS, PHILADELPHIA, PA, US, vol. 12, no. 3, 1 January 2002 (2002-01-01), pages 259-270, XP001133467, ISSN: 0898-2104, DOI: 10.1081/LPR-120014761 * abstract; figure 1 * * page 261, paragraph 2 * * page 262, paragraph 3; figure 2 * | 1-15 | |

The present search report has been drawn up for all claims

| Place of search | Date of completion of the search | Examiner |
|---|---|---|
| The Hague | 19 October 2020 | Schwald, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 17 6366

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2020

| Patent document cited in search report | | Publication date | Patent family member(s) | | Publication date |
|---|---|---|---|---|---|
| WO 2014152200 | A1 | 25-09-2014 | AU 2014240013 | A1 | 13-08-2015 |
| | | | CA 2898047 | A1 | 25-09-2014 |
| | | | CN 105188685 | A | 23-12-2015 |
| | | | EP 2968193 | A1 | 20-01-2016 |
| | | | JP 6450742 | B2 | 09-01-2019 |
| | | | JP 2016517423 | A | 16-06-2016 |
| | | | KR 20150127580 | A | 17-11-2015 |
| | | | US 2014348900 | A1 | 27-11-2014 |
| | | | US 2018000735 | A1 | 04-01-2018 |
| | | | WO 2014152200 | A1 | 25-09-2014 |
| US 2005112184 | A1 | 26-05-2005 | NONE | | |
| WO 2016141161 | A1 | 09-09-2016 | CA 2977397 | A1 | 09-09-2016 |
| | | | CN 107530291 | A | 02-01-2018 |
| | | | EP 3265063 | A1 | 10-01-2018 |
| | | | JP 2018507227 | A | 15-03-2018 |
| | | | US 2016256387 | A1 | 08-09-2016 |
| | | | WO 2016141161 | A1 | 09-09-2016 |
| US 2015209283 | A1 | 30-07-2015 | AU 2009305639 | A1 | 22-04-2010 |
| | | | AU 2016222371 | A1 | 15-09-2016 |
| | | | AU 2018204605 | A1 | 12-07-2018 |
| | | | BR PI0920171 | A2 | 12-07-2016 |
| | | | CA 2739046 | A1 | 22-04-2010 |
| | | | CN 102231979 | A | 02-11-2011 |
| | | | CN 104382853 | A | 04-03-2015 |
| | | | EP 2349210 | A2 | 03-08-2011 |
| | | | EP 2902013 | A1 | 05-08-2015 |
| | | | JP 6126072 | B2 | 10-05-2017 |
| | | | JP 2012505913 | A | 08-03-2012 |
| | | | JP 2015096529 | A | 21-05-2015 |
| | | | KR 20110071017 | A | 27-06-2011 |
| | | | SG 196818 | A1 | 13-02-2014 |
| | | | US 2010112042 | A1 | 06-05-2010 |
| | | | US 2013039970 | A1 | 14-02-2013 |
| | | | US 2015209283 | A1 | 30-07-2015 |
| | | | WO 2010045512 | A2 | 22-04-2010 |
| | | | ZA 201102319 | B | 26-09-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2395978 B1
- EP 1165224 B1
- EP 2550092 A1
- DE 102016101232 A1
- WO 2018234217 A1

**Non-patent literature cited in the description**

- **CHRAI S. ; MURARI R. ; AHMAD I.** Liposomes (a Review), Part Two: Drug Delivery Systems. *BioPharm,* 2002, 40-49
- **HONG M. ; LIM S. ; LEE M. ; KIM Y. ; KIM C.** Prolonged Blood Circulation of Methotrexate by Modulation of Liposomal Composition. *Drug Delivery,* 2001, vol. 1 (8), 231-237
- Liposomes: Design and Manufacturing. **PATIL, S. D. ; BURGESS, D. J.** Injectable Dispersed Systems. Informa Healthcare, 2005, vol. 20053164, 249-303